Europäisches Patentamt

European Patent Office

Office européen des brevets

(11) Publication number: **0 312 612 B1**

# EUROPEAN PATENT SPECIFICATION

(45) Date of publication of patent specification: **24.03.93**

(51) Int. Cl.5: **A23L 2/38**, A23L 1/48,
A61K 31/195, A23L 1/305

(21) Application number: **88903936.8**

(22) Date of filing: **27.04.88**

(86) International application number:
**PCT/JP88/00421**

(87) International publication number:
**WO 88/08259 (03.11.88 88/24)**

The file contains technical information submitted
after the application was filed and not included in
this specification

(54) **NUTRITIVE EMULSION.**

(30) Priority: **01.05.87 JP 109577/87**

(43) Date of publication of application:
**26.04.89 Bulletin 89/17**

(45) Publication of the grant of the patent:
**24.03.93 Bulletin 93/12**

(84) Designated Contracting States:
**FR**

(56) References cited:
**JP-A- 6 158 560**
**JP-A-58 126 767**
**JP-B- 5 526 816**

(73) Proprietor: **OTSUKA PHARMACEUTICAL CO.,
LTD.**
**9, Kandatsukasacho 2-chome
Chiyoda-ku Tokyo 101(JP)**

(72) Inventor: **IWANO, Seiji 2FE, Shinsei Bldg.**

20-2, Aza Ebisumae-higashi Minamihama
Muya-cho
Naruto-shi Tokushima 567(JP)
Inventor: **KADOWAKI, Tomonori**
**1119-1, Kizu Muya-cho**
Naruto-shi Tokushima 772(JP)
Inventor: **NAKAMURA, Toshio Takeuchi Man-
shon**
**72-2, Aza Motosu Nakamura Kitajima-cho**
Itano-gun Tokushima 771-02(JP)
Inventor: **SOGAWA, Yoshiro**
**3-8-48, Sumiyoshi Tokushima-shi**
Tokushima 770(JP)
Inventor: **MABUCHI, Hideko**
**307, Okamoto-haitsu 3-31-5, Sanjima-cho**
Kitajo Tokushima-shi Tokushima 770(JP)
Inventor: **SAITO, Yuichi**
**28-31, Aza Nakazao Shinkirai Kitajima-cho**
Itano-gun Tokushima 771-02(JP)

(74) Representative: **Ores, Irène et al**
**CABINET ORES 6, Avenue de Messine**
**F-75008 Paris (FR)**

## Description

Technical Field

The present invention relates to a novel nutritive emulsion.

Prior Art

Patients of a disease such as gastrointestinal disease and those having a post-operative early-stage stress are likely to have difficulty in taking, or to be unable to take, orally a nutrient source such as meals. Further, such patients experience marked destruction (catabolism) of endogenous protein, reduction in function of treatment (anabolism) of amino acid or the like, and are found abnormal in utilization of endogenous amino acids and other nutrients.

Researches have been heretofore conducted in an attempt for these patients to meet the nutrition requirements (feeding, nutrient control and nitrogen equilibrium maintenance) and to achieve the improvement in preservation of physical strength and the recovery of physical function. The investigations so far made resulted in development of amino acid parenteral solutions, fat emulsions and other nutritive acents for feeding amino acids and other nutrients. An ideal nutritive agent is a comprehensive nutritive agent containing amino acids, lipids, sugars, vitamins, minerals and the like which are essential to human body. However, such ideal comprehensive nutritive agent has yet to be developed and the development thereof is now strongly desired in the art.

For instance, the Japanese patent Application A 61 58560 describes an edible composition containing saccharides, protein (or its hydrolysate), lipids, minerals, vitamins, and higher unsaturated fatty acids. However, such a composition is conceived in order to be administered to patients suffering from cardiovascular disease, and not in order to supply essential nutriments to patients unable to take food orally.

We performed extensive research to provide the comprehensive nutritive agent currently desired in the art. Our research led to successful development of a novel nutritive emulsion which contains sufficient amounts of amino acids and lipids, which is easy to manufacture, free of bacterial contamination problem, oustanding in stability in preparation form, capable of course, of supplying a sufficient nutrition and readily miscible with sugars, vitamins, minerals and the like when used, and which thus provides an ideal comprehensive nutritive agent. In this way, we have accomplished the present invention.

Disclosure of the invention

The present invention relates to a nutritive emulsion containing, as essential components, an amino acid preparation, a lipid, water and an emulsifier, which is characterized in that the emulsion comprises 4 to 15% by weight of the amino acid preparation having the amino acid composition shown in Table I below and calculated as free amino acids based on the total amino acids, in which the amount of each amino acid is expressed in percentage by weight, 3 to 12% by weight of a mixture of medium-chain fatty acid triglyceride (MCT) and long-chain fatty acid triglyceride (LCT) as the lipid, 71.5 to 93% by weight of water, and 0.3 to 3.0% by weight of the emulsifier having an HLB of at least 13 and selected from the group consisting of sucrose fatty ester, glycerin fatty acid ester, polyoxyethylene sorbitan fatty acid ester and an emulsifier mixture of said emulsifier and soybean lecithin and in that the emulsifier has a pH of 6.5 to 8.5 :

Table 1

| Amino acid | Amount (wt%) |
|---|---|
| L-isoleucine | 4.5-13.4 |
| L-leucine | 6.7-20.1 |
| L-lysine | 3.8-15.2 |
| L-metionine | 3.0-16.4 |
| L-phenylalanine | 3.1-16.4 |
| L-threonine | 3.2- 9.6 |
| L-tryptophan | 0.9- 3.7 |
| L-valine | 5.2-15.6 |
| L-alanine | 0 - 9.6 |
| L-arginine | 0 -12.6 |
| L-aspartic acid | 0 -12.2 |
| L-glutamic acid | 0 -12.9 |
| L-glutamine | 0 -16.2 |
| Aminoacetic acid (glycine) | 0 - 4.1 |
| L-histidine | 0 - 6.2 |
| L-proline | 0 - 5.3 |
| L-serine | 0 - 9.7 |
| L-tyrosine | 0 - 0.9 |

The nutritive emulsion of the present invention is characterized by containing various amino acids and lipids in the emulsion, being easy to manufacture, free of bacterial contamination issue, able to supply the patient with sufficient amounts of amino acids and lipids, especially high in fat content and excellent in physiological properties, eliminating the need to add a fat before administration, and in long-term storage stability (emulsion stability).

The nutritive emulsion of the invention is easily miscible with sugars, vitamins, minerals and the like at the time when the emulsion is used. Even the mixing substantially does not reduce the stability and thus an ideal comprehensive nutritive agent can be provided.

When the nutritive-emulsion of the invention is rectally or orally administered to particularly a patient of disease such as gastrointestinal disease or a patient having a post-operative early-stage stress, the patient's nutrition requirements (including feeding, nutrient control and nitrogen equilibrium maintenance) can be satisfactorily met, and the improvement in preservation of patient's physical strength and recovery of patient's physical function can be effectively achieved.

The nutritive emulsion of the invention is prepared by emulsification of the above-specified amounts of amino acid preparation and lipid with a specific emulsifier and characterized by a emulsified form with a excellent emulsion stability. This feature gives the contemplated remarkable results, particularly long-lasting stability and supply of high-calory nutrients. The nutritive emulsion with such composition is unknown and undisclosed.

The amino acid preparation as one of essential components of the nutritive emulsion according to the invention can be those having the same composition as that of various amino acid preparations conventionally investigated and developed in medical area in order to feed a nutrient and can be those of the composition which we have developed on the basis of these conventional amino acid preparations. These amino acid preparations generally contain eight essential amino acids, i.e. isoleucine (Ile), leucine (Leu), valine (Val), methionine (Met), phenylalanine (Phe), tryptophan (Trp), lysine (Lys) and threonine (Thr), and, when required, may further contain at least one subessential or nonessential amino acid selected from cysteine (Cys), tyrosine (Tyr), histidine (His), alanine (Ala), arginine (Arg), aspartic acid (Asp), glutamic acid (Glu), glycine (Gly), proline (Pro), serine (Ser), ornithine (Orn) and the like.

Among the preferred amino acid preparations in the invention are those containing branched-chain amino acids in combined amount (Ile + Leu + Val) of 20 to 45% by weight, preferably 30 to 40% by weight, based on the total amino acids. Preferred other examples of amino acid preparations include those containing eight essential amino acids and at least one amino acid selected from the above-exemplified subessential and nonessential amino acids, the total amount of the eight essential amino acids being 40 to 80% by weight, more preferably 50 to 70% by weight, based on the total amino acids.

A preferable composition of the amino acid preparation is shown below in Table 2.

4

Table 2

| Amino acid | Amount (wt%) |
|---|---|
| L-isoleucine | 4.5-13.4 |
| L-leucine | 6.7-20.1 |
| L-lysine | 3.8-11.6 |
| L-methionine | 3.3- 7.7 |
| L-phenylalanine | 3.1- 9.5 |
| L-threonine | 3.2- 9.6 |
| L-tryptophan | 0.9- 2.7 |
| L-valine | 5.2-15.6 |
| L-alanine | 3.2- 9.6 |
| L-arginine | 0.9- 2.7 |
| L-aspartic acid | 1.6- 5.0 |
| L-glutamic acid | 4.3-12.9 |
| aminoacetic acid (glycine) | 1.3- 4.1 |
| L-histidine | 1.0- 3.2 |
| L-proline | 1.5- 4.5 |
| L-serine | 2.7- 8.1 |

The most threpsologically preferable composition of the amino acid preparation is similarly shown below in Table 3.

Table 3

| Amino acid | Amount (wt%) |
|---|---|
| L-isoleucine | 8.0-13.4 |
| L-leucine | 12.5-20.1 |
| L-lysine | 6.9- 8.5 |
| L-methionine | 4.6- 5.6 |
| L-phenylalanine | 5.6- 6.9 |
| L-threonine | 3.2- 7.0 |
| L-tryptophan | 1.6- 2.0 |
| L-valine | 5.2-11.4 |
| L-alanine | 5.8- 7.0 |
| L-arginine | 7.6- 9.2 |
| L-aspartic acid | 3.0- 3.6 |
| L-glutamic acid | 7.7- 9.5 |
| aminoacetic acid (glycine) | 2.4- 3.0 |
| L-histidine | 1.9- 2.3 |
| L-proline | 2.7- 3.3 |
| L-serine | 4.9- 5.9 |

The amino acids which constitute the amino acid preparation having the composition in said range are preferably purely crystalline amino acids. These amino acids are generally used in free acid form but are not especially limited to this form. The amino acids may take various water-soluble forms, which include pharmaceutically acceptable salts including metal salts such as sodium salt and potassium salt, mineral acid salts such as hydrochloride and sulfate and organic acid salts such as acetate, lactate and malate; and esters, N-acyl derivatives, peptides and the like which are converted into free amino acids by hydrolysis in vivo. Especially the use of amino acids in the form of peptide results in lover osmotic pressure of the obtained amino acid preparation than in free acid form, advantageously assuring the obviation of diarrhea problem which is apt to arise in the patient administered with the obtained amino acid preparation. Examples of salts are L-lysine monohydrochloride, L-lysine acetate, L-lysine malate, L-arginine monohydrochloride, monosodium L-aspartate monohydrate, monosodium L-glutamate monohydrate, L-histidine monohydrochloride monohydrate and the like. Examples of esters are L-methionine methyl ester, L-methionine ethyl ester and the like. Examples of N-acyl derivatives are N-lower alkanoyl derivatives such as N-acetyl-L-tyrosine, N-acetyl-L-proline and N-acetyl-L-tryptophan. Examples of peptides are L-alanyl-L-phenylalanine, L-alanyl-L-valine, L-alanyl-glycine, L-leucyl-L-phenylalanine, L-leucyl-L-alanine, L-leucyl-L-valine, L-leucyl-L-leucine, L-leucyl-glycine, glycyl-L-phenylalanine, glycyl-L-alanine, glycyl-L-leucine, L-tryptophyl-L-alanine, L-tryptophyl-L-leucine, L-tryprophyl-L-phenylalanine, L-tryptophyl-glycine, L-tyrosyl-L-tyrosine, L-alanyl-L-tyrosine and like dipeptides; glycyl-L-leucyl-L-leucine, glycyl-L-alanyl-L-alanine, L-alanyl-L-phenylalanyl-L-valine, L-tryptophyl-glycyl-glycine, L-tryptophyl-glycyl-L-alanine, glycylglycyl-glycine and like tripeptides; glycyl-glycyl-glycyl-glycine and like tetrapeptides; glycyl-glycyl-glycyl-glycyl-glycine and like pentapeptides; etc.

Further, L-cysteine may be partially or wholly replaced by L-cystine and/or L-methionine, and L-tyrosine may be partially or wholly replaced by L-phenylalanine. The amino acids constituting the amino acid preparation of the invention may be also partially replaced by water-soluble gelatin, casein hydrolyzate or the like. If the amino acids are used in the form other than free form, the amount of amino acids calculated as free amino acid is determined to fall within the above-defined range.

The lipid which is among the other essential components for the nutritive emulsion of the invention can be any of various oils conventionally used as an energy source for supply of nutrients. Examples of useful lipids are medium-chain fatty acid triglycerides (MCT) characterized by ease in absorption and in combustion and rarity in accumulation, and long-chain fatty acid triglycerides (LCT) serving as the source of essential fatty acids. Preferable as MCT are triglycerides of saturated or unsaturated fatty acids having 8 to 10 carbon atoms. Usable as LCT are those commonly used such as soybean oil, cotton seed oil and sesame oil. Concomitant use of MCT and LCT as lipids in the invention is generally preferable. Suitable proportions are 20 to 90 % by weight, preferably 50 to 90 % by weight, of MCT and 10 to 80% by weight, preferably 10 to 50% by weight, of LCT.

The nutritive emulsion of the invention is prepared by emulsifying the amino acid preparation and the lipid with a specific emulsifier to disperse the components in water. The emulsifier to be used in the invention is critically selected from sucrose fatty acid ester, glycerin fatty acid ester, polyoxyethylene glycerin fatty acid ester and polyoxyethylene sorbitan fatty acid ester which each have a HLB of at least 13 and a mixture of the emulsifier and soybean lecithin.

Emulsifiers useful in the invention are those having a HLB of 13 or more, preferably 13 to 15 and selected from sucrose fatty acid ester, glycerin fatty acid ester, polyoxyethylene glycerin fatty acid ester and polyoxyethylene sorbitan fatty acid ester, as described above. These emulsifiers are usable singly or at least two of them can be used in mixture in a total HLB of at least 13, preferably 13 to 15. In other words, the term "emulsifier having a HLB of at least 13" used herein is intended to encompass an emulsifier mixture having a HLB of at least 13. The emulsifier mixture includes a mixture of sucrose fatty acid esters, a mixture of glycerin fatty acid esters, a mixture of polyoxyethylene glycerin fatty acid esters, a mixture of polyoxyethylene sorbitan fatty acid esters and a mixture of these esters. Examples of the ester composing the emulsifier are sucrose ester, glycerin ester, polyoxyethylene glycerin ester and polyoxyethylene sorbitan ester of $C_{12}$-$C_{20}$ fatty acid such as stearic acid and palmitic acid. More specifically, the sucrose fatty acid ester which can be used singly includes "DK-F-140" (HLB 13.5, product of Daiichi Kogyo Pharmaceutical, Co., Ltd.), "DK-F-160" (HLB 15.0, product of the same company), "S-1570" (HLB 15.0, product of Mitsubishi Kasei Shokuhin K.K.), etc. The sucrose fatty acid ester which can be used in mixture includes "DK-F-110" (HLB 11.0, product of Daiichi Kogyo Pharmaceutical, Co., Ltd.), "DK-F-90" (HLB 9.5, product of the same company), etc. Examples of glycerin fatty acid ester usable singly are "Decaglyn 1-M" (HLB 14, product of Nikko Chemicals Co., Ltd.), "Decaglyn 1-L" (HLB 15.5, product of the same company), "Hexaglyn 1-L" (HLB 13, product of the same company), etc. Examples of polyoxyethylene glycerin fatty acid ester are "TMGS-15" (HLB 13.5, product of Nikko Chemicals, Co., Ltd.), "TGSO-215" (HLB 13.5, product of the same company), etc. Examples of polyoxyethylene sorbitan fatty acid ester are "TO-10" (HLB 15.0, product of Nikko Chemicals Co., Ltd.), "Rheodol TW-O120" (HLB 15.0, product of Kao Co., Ltd.), etc.

The emulsifier of at least 13 in HLB may be used conjointly with other emulsifiers, e.g. polyoxyethylene hydrogenated castor oil, polyoxyethylene castor oil, polyoxyethylene sorbitan fatty acid ester, etc.

A mixture of an emulsifier of at least 13 in HLB and soybean lecithin can be also used. Examples of soybean lecithins useful in the invention can be any of various kinds of those generally containing a mixture of phosphatidyl choline, phosphatidyl ethanolamine and phosphatidyl inositol as phospholipids. Among them, a powder soybean lecithin, particularly a powder containing a high-purity lecithin, is preferable. Examples of preferable powder of soybean lecithin include commercially available lecithin, e.g., lecithin powder containing 20 to 25% of phosphatidyl choline and 98% of lecithin("EPIKURON 100 P", Nihon Siber Hegner K.K.), lecithin powder containing 30% of phosphatidyl choline and 98% of lecithin ("EPIKURON 130 P", Nihon Siber Hegner K.K.), etc.

While there is no specific limitation on the ratio of the soybean lecithin to the emulsifier with a HLB of at least 13 in the emulsifier mixture, generally 25% by weight or less, preferably 10 to 25% by weight, of soybean lecithin is used conjointly with 75% by weight or more, preferably 75 to 90% by weight, of emulsifier mixture with a HLB of at least 13.

The nutritive emulsion of the invention comprises 4 to 15% by weight, preferably 8 to 10% by weight , of amino acid preparation, 3 to 12% by weight, preferably 6 to 10% by weight, of a lipid, 71.5 to 93% by weight, preferably 80 to 85% by weight, of water, and 0.3 to 3.0% by weight, preferably 0.6 to 2.0% by weight, of an emulsifier. The use of components in these proportions affords the desired results of the invention, particularly the desired nutritive emulsion with excellent emulsion stability.

The soybean lecithin which may be used as one of emulsifiers in the invention may be classified as a lipid in view of its properties, but is not to be calculated as a lipid in determining the amounts of components in the nutritive emulsion of the invention.

The desired nutritive emulsion comprising the above components can be prepared by usual methods. A preferable methods comprises adding a lipid or a mixture of a lipid and soybean lecithin to a mixture of water and an emulsifier of 13 or more in HLB; subjecting the mixture to preliminary emulsification (first pre-emulsification); adding an aqueous solution of an amino acid preparation to the coarse emulsified liquid; and subjecting the mixture to emulsification (second pre-emulsification and fine emulsification). The aqueous solution of amino acid preparation can be prepared in the same manner as done in producing a usual amino acid parenteral solution or the like, for example, by dissolving the amino acids or their derivatives in distilled water or the like useful for injection and adding, when required, a stabilizer such as sodium sulfite, sodium hydrogensulfite, sodium pyrosulfite or sodium thiosulfate, a pH adjustor such as hydrochloric acid, acetic acid, lactic acid, malic acid, citric acid, sodium hydroxide or potassium hydroxide, and other conventional additives useful for amino acid injection, e.g., water-soluble vitamins, lipid-soluble vitamins, minerals such as inorganic salts and preservatives such as sodium ascorbate, followed by sterilization with heating or filtration. The components can be mixed at ambient temperature, preferably at a slightly higher temperature (55 to 70°C) The nutritive emulsion of the invention can be prepared also by diluting the coarse emulsified liquid with water, fine emulsifying the dilution and mixing the resulting emulsion with an aqueous solution of amino acid preparation. The preliminary emulsification and fine emulsification can be done in a usual manner by either a completely passing method or circulation method using a proper emulsifying machine such as a homomixer (5000 to 7000 rpm) or a high-pressure homogenizer 421 842 to 562 456 $Pa.s^2. m^{-1}$ (6000 to 8000 psi). The emulsion prepared by the fine emulsification may be filtered in a conventional manner, dividedly placed into containers and sterilized, giving a desired nutritive emulsion.

In this way, the nutritive emulsion of the invention can be prepared. The emulsion has a pH of 6.5 to 8.5 and is not only stable as a pharmaceutical preparation but also outstanding in emulsion stability. The emulsion of the invention contains oil particles small and uniform in particle size and has such high storage stability as to sustain the original stability for a long term, generally for 2 months or longer at 40°C, 75% RH and for 1 year or longer at room temperature. The emulsion can be orally or rectally administered to a patient at a dose of 700 to 1000 ml/day/adult which is sufficient to give 700 to 1000 Kcal per day.

The nutritive emulsion of the invention may be made into a high-calory comprehensive nutritive agent by adding a suitable kind of sugar and other additives such as vitamins, minerals (electrolytes) and trace elements to the nutritive emulsion when used. The nutritive emulsion of the invention has the feature that substantially neither the emulsion form nor the emulsion stability is impaired by use of such additives.

Examples of sugars which can be added to the nutritive emulsion of the invention are monosaccharides such as glucose, fructose, xylitol and sorbitol and disaccharides such as maltose and sucrose, known to be usually used as an energy source, and polysaccharides such as dextrin and cyclodextrin, among which disaccharides and polysaccharides are preferable.

Useful vitamins include a wide variety of water-soluble or lipid-soluble vitamins such as retinol palmitate, bisbentiamine, riboflavin, pyridoxine hydrochloride, cyanocobalamin, sodium ascorbate, cholecalciferol, nicotinamide, calcium pantothenate, folic acid, biotin, choline bitartarate, etc.

Illustrative of electrolytes (mineral) and trace elements are those commonly used such as sodium chloride, sodium acetate, magnesium sulfate, magnesium chloride, calcium chloride, dipotassium hydrogenphosphate, sodium dihydrogenphosphate, calcium glycerophosphate, sodium ferrous citrate, manganese sulfate, copper sulfate, zinc sulfate, sodium iodide, potassium sorbate, zinc, manganese, copper, iodine, cobalt and the like.

The amounts of the additives used are the same as those of conventional ones and can be properly determined according to the patient's demand. When phytonadione is used as vitamin, it is suitable to incorporate the same into the nutritive emulsion as a lipid-soluble vitamin.

The nutritive emulsion of the invention may further contain dietary fibers such as corn fiber, yellow bee fiber, polydextrose, powdered cellulose, guar gum or the like in order to provide nutrition.

It is desirable that suitable amounts of additives to be used be mixed together to form a powder mixture. The powder is preferably added to the nutritive emulsion of the invention before administration, whereby a comprehensive nutritive agent is formed. The comprehensive nutritive agent, if required, may also contain xanthan gum, hydroxypropyl methyl cellulose or the like as a viscosity promoter for emulsion stability to adjust the viscosity up to 0.05 Pa.S (50 cp). This addition may further improve the emulsion stability.

The comprehensive nutritive agent, i.e., the nutritive emulsion of the invention combined with powdery additives, is useful as a high-calory comprehensive nutritive agent. Suitably the amino acid preparation, lipid and sugar in the agent are used in an approximate ratio of 10-20 : 20-35 : 45-70 by percent calorie and that in veiw of stability of vitamins and the like to be contained, the agent have a pH of 5 to 6. The comprehensive nutritive agent remains stable for at least 12 hours after mixing. Particularly, the agent having incorporated therein an amino acid preparation containing a peptide assures freedom from the possibility of causing diarrhea or like side effects which otherwise would be likely to occur in the patient treated with conventional nutritive emulsions, and thus its administration results in unlikelihood to raise such side effect problem in the patient. The comprehensive nutritive agent can be provided as a high-calory nutritive agent giving 0.5 to 2.5 Kcal/ml/day/adult, preferably 2 Kcal/ml/day/adult. It is suitable that the agent be administered at a daily dose sufficient to give 1800 to 2400 Kcal. The dose can be suitably varied, of course, depending on the symptoms, nutriture, age, weight and other factors of the patient.

Examples

The present invention will be described below in more detail with reference to the following Examples illustrating the preparation of nutritive emulsions according to the invention and Test Examples.

Example 1

| | |
|---|---|
| Total amino acids (free) | 97 g (90.1) |
| L-isoleucine | 8.0 |
| L-leucine | 12.1 |
| L-lysine monohydrochloride | 8.6 (6.9) |
| L-methionine | 4.6 |
| L-phenylalanine | 5.7 |
| L-threonine | 5.8 |

8

| | |
|---|---|
| L-tryptophan | 1.6 |
| L-valine | 9.4 |
| L-alanine | 5.8 |
| L-arginine monohydrochloride | 9.2 (7.6) |
| monosodium L-aspartate.$H_2O$ | 3.9 (3.0) |
| monosodium L-glutamate.$H_2O$ | 9.8 (7.7) |
| aminoacetic acid (glycine) | 2.4 |
| L-histidine monohydrochloride.$H_2O$ | 2.5 (1.9) |
| L-proline | 2.7 |
| L-serine | 4.9 |
| Total lipids | 80 g |
| medium-chain fatty acid triglyceride | 68.0 |
| soybean oil | 12.0 |
| Total vitamins | 100 µg |
| phytonadione | 100 |
| Total minerals | 1.3 g |
| potassium hydroxide | 1.3 |
| Total emulsifiers | 7.6 g |
| purified soybean lecithin[1] | 1.2 |
| sucrose fatty acid ester[2] | 6.4 |
| Purified water | about 860 ml (pH 7.5) |

(Note)

1) "EPIKURON P100" (Nihon Siber Eegner K.K.)

2) A mixture (HLB 13.5) of "DK-F-160" and "DK-F-90" (both

products of Daiichi Kogyo Seiyaku Co., Ltd.) in a ratio of

3 to 1 by weight

The same products as those above in 1) and 2) were used in subsequent examples.

Using the amino acids, lipids, vitamins, minerals, pH adjustor (potassium hydroxide) in the above amounts, the nutritive emulsion of the invention was prepared as follows.

9

The sucrose fatty acid ester was dissolved in 150 ml of water at 60°C. A mixture of medium-chain fatty acid triglyceride, soybean oil and purified soybean lecithin was added to the solution at 60°C. At the same temperature, the mixture was subjected to a first pre-emulsification using "T.K. Homomixer" (product of Tokushu Kika Kogyo Co., Ltd.) at 6000 rpm for 5 minutes. An amino acid preparation prepared by mixing 710 ml of water, amino acids and potassium hydroxide at 60°C was added to the coarse emulsified liquid. The mixture was subjected to a second pre-emulsification under the same conditions as above (60°C, 6000 rpm, 5 minutes). Finally the coarse emulsified liquid was caused to undergo a fine emulsification with a high pressure homogenizer (60-70°C, 8000 psi, 6 times passage). Thereafter the obtained emulsion was filtered (a filter of 1.2 $\mu$m mesh size), placed into containers (after nitrogen gas replacement) and sterilized (116°C, 30 minutes), giving the nutritive emulsion of the present invention in a total amount of 1000 ml (1039 g).

Example 2

| | |
|---|---|
| Total amino acids (free) | 98.1 g (92.9) |
| L-isoleucine | 9.4 |
| L-leucine | 14.6 |
| L-lysine monohydrochloride | 16.9 (13.5) |
| L-methionine | 14.6 |
| L-phenylalanine | 14.6 |
| L-threonine | 6.7 |
| L-tryptophan | 3.3 |
| L-valine | 10.7 |
| L-histidine monohydrochloride $H_2O$ | 7.3 (5.5) |
| Total lipids | 80 g |
| medium-chain fatty acid triglyceride | 68.0 |
| soybean oil | 12.0 |
| Total vitamins | 100 $\mu$g |
| phytonadione | 100 |
| Total emuilsifiers | 7.6 g |
| purified soybean lecithin[1] | 1.2 |
| sucrose fatty acid ester[2] | 6.4 |
| Purified water | about 860 ml (pH 7.5) |

In the same manner as in Example 1, an aqueous solution of sucrose fatty acid ester and a mixture of a lipid and soybean lecithin were subjected to a first pre-emulsification with a homomixer. Then, amino acids were added and the solution was adjusted to a pH of 7.5 with a 8N potassium hydroxide aqueous solution. Water was added in an amount to bring the total amount to 1000 ml. The resulting solution was subjected to a second pre-emulsification and fine emulsification, filtered, placed into containers and sterilized, giving the nutritive emulsion of the invention.

Example 3

| | |
|---|---|
| Total amino acids (free) | 107.7 g (99.9) |
| L-isoleucine | 8.9 |
| L-leucine | 13.4 |
| L-lysine monohydrochloride | 9.6 (7.7) |
| L-methionine | 5.1 |
| L-phenylalanine | 6.3 |
| L-threonine | 6.4 |
| L-tryptophan | 1.8 |
| L-valine | 10.4 |
| L-alanine | 6.4 |
| L-arginine monohydrochloride | 10.2 (8.4) |
| monosodium L-aspartate.$H_2O$ | 4.3 (3.3) |
| monosodium L-glutamate.$H_2O$ | 11.0 (8.6) |
| aminoacetic acid (glycine) | 2.7 |
| L-histidine monohydrochloride $H_2O$ | 2.8 (2.1) |
| L-proline | 3.0 |
| L-serine | 5.4 |
| Total lipids | 80 g |
| medium-chain fatty acid triglyceride | 68.0 |
| soybean oil | 12.0 |
| Total vitamins | 100 $\mu$g |
| phytonadione | 100 |
| Total emulsifiers | 7.6 g |
| purified soybean lecithin[1] | 1.2 |
| sucrose fatty acid ester[2] | 6.4 |
| Purified water | about 860 ml (pH 7.5) |

In the same manner as in Example 1, an aqueous solution of sucrose fatty acid ester and a mixture of a lipid and soybean lecithin were subjected to a first pre-emulsification with a homomixer. Then, amino acids were added and the solution was adjusted to a pH of 7.5 with a 8N potassium hydroxide aqueous solution. Water was added in an amount to bring the total amount to 1000 ml. The resulting solution was subjected to a second pre-emulsification and fine emulsification, filtered, placed into containers and sterilized, giving the nutritive emulsion of the invention.

Example 4

| | |
|---|---|
| Total amino acids (free) | 97 g (90.1) |
| L-isoleucine | 8.0 |
| L-leucine | 12.1 |
| L-lysine monohydrochloride | 8.6 (6.9) |
| L-methionine | 4.6 |
| L-phenylalanine | 5.7 |
| L-threonine | 5.8 |
| L-tryptophan | 1.6 |
| L-valine | 9.4 |
| L-alanine | 5.8 |
| L-arginine monohydrochlorde | 9.2 (7.6) |
| monosodium L-aspartate.$H_2O$ | 3.9 (3.0) |
| monosodium L-glutamate.$H_2O$ | 9.8 (7.7) |
| aminoacetic acid (glycine) | 2.4 |
| L-histidine monohydrochloride $H_2O$ | 2.5 (1.9) |
| L-proline | 2.7 |
| L-serine | 4.9 |
| Total lipids | 80 g |
| medium-chain fatty acid triglyceride | 68.0 |
| soybean oil | 12.0 |
| Total emulsifiers | 7.6 g |
| purified soybean lecithin[1] | 1.2 |
| sucrose fatty acid ester[2] | 6.4 |
| Purified water | about 860 ml (pH 7.5) |

In the same manner as in Example 1, an aqueous solution of sucrose fatty acid ester and a mixture of a lipid and soybean lecithin were subjected to a first pre-emulsification with a homomixer. Then, amino acids were added and the solution was adjusted to a pH of 7.5 with a 8N potassium hydroxide aqueous solution. Water was added in an amount to bring the total amount to 1000 ml. The resulting solution was subjected to a second pre-emulsification and fine emulsification, filtered, placed into containers and sterilized, giving the nutritive emulsion or the invention.

The nutritive emulsions each having a pH of 6.9 and a pH of 7.9 were prepared in the same manner as above except that the amount of 8N potassium hydroxide aqueous solution was changed.

Example 5

| | |
|---|---|
| Total amino acids (free) | 97 g (90.1) |
| L-isoleucine | 8.0 |
| L-leucine | 12.1 |
| L-lysine monohydrochloride | 8.6 (6.9) |
| L-methionine | 4.6 |
| L-phenylalanine | 5.7 |
| L-threonine | 5.8 |
| L-tryptophan | 1.6 |
| L-valine | 9.4 |
| L-alanine | 5.8 |
| L-arginine monohydrochloride | 9.2 (7.6) |
| monosodium L-aspartate.$H_2O$ | 3.9 (3.0) |
| monosodium L-glutamate.$H_2O$ | 9.8 (7.7) |
| aminoacetic acid (glycine) | 2.4 |
| L-histidine monohydrochloride $H_2O$ | 2.5 (1.9) |
| L-proline | 2.7 |
| L-serine | 4.9 |
| Total lipids | 80 g |
| medium-chain fatty acid triglyceride | 68.0 |
| soybean oil | 12.0 |
| Total emulsifiers | 7.0 g |
| purified soybean lecithin[1] | 1.1 |
| sucrose fatty acid ester[2] | 5.9 |
| Purified water | about 860 ml (pH 7.5) |

In the same manner as in Example 1, an aqueous solution of sucrose fatty acid ester and a mixture of a lipid and soybean lecithin were subjected to a first pre-emulsification with a homomixer. Then, amino acids were added and the solution was adjusted to a pH of 7.5 with a 8N potassium hydroxide aqueous solution. Water was added in an amount to bring the total amount to 1000 ml. The resulting solution was subjected to a second pre-emulsification and fine emulsification, filtered, placed into containers and sterilized, giving the nutritive emulsion or the invention.

Example 6

| | |
|---|---|
| Total amino acids (free) | 97 g (93.1) |
| L-isoleucine | 8.0 |
| L-leucine | 12.1 |
| L-lysine monohydrochloride | 8.6 (6.9) |
| L-methionine | 4.6 |
| L-phenylalanine | 5.7 |
| L-threonine | 5.8 |
| L-tryptophan | 1.6 |
| L-valine | 9.4 |
| L-alanine | 5.8 |
| L-arginine monohydrochloride | 9.2 (7.6) |
| monosodium L-aspartate.$H_2O$ | 3.9 (3.0) |
| monosodium L-glutamate.$H_2O$ | 9.8 (7.7) |
| aminoacetic acid (glycine) | 2.4 |
| L-histidine monohydrochloride $H_2O$ | 2.5 (1.9) |
| L-proline | 2.7 |
| L-serine | 4.9 |
| Total lipids | 104 g |
| medium-chain fatty acid triglyceride | 88.4 |
| soybean oil | 15.6 |
| Total emulsifiers | 9.7 g |
| purified soybean lecithin[1] | 1.5 |
| sucrose fatty acid ester[2] | 8.2 |
| Purified water | about 860 ml (pH 7.5) |

In the same manner as in Example 1, an aqueous solution of sucrose fatty acid ester and a mixture of a lipid and soybean lecithin were subjected to a first pre-emulsification with a homomixer. Then, amino acids were added and the solution was adjusted to a pH of 7.5 with a 8N potassium hydroxide aqueous solution. Water was added in an amount to bring the total amount to 1000 ml. The resulting solution was subjected to a second pre-emulsification and fine emulsification, filtered, placed into containers and sterilized, giving the nutritive emulsion of the invention.

14

Example 7

| Total amino acids (free) | 111.8 g (104.9) |
|---|---|
| L-isoleucine | 12.0 |
| L-leucine | 18.2 |
| L-lysine monohydrochloride | 8.6 (6.9) |
| L-methionine | 4.6 |
| L-phenylalanine | 5.7 |
| L-threonine | 5.8 |
| L-tryptophan | 1.6 |
| L-valine | 14.1 |
| L-alanine | 5.8 |
| L-arginine monohydrochloride | 9.2 (7.6) |
| monosodium L-aspartate.$H_2O$ | 3.9 (3.0) |
| monosodium L-glutamate.$H_2O$ | 9.8 (7.7) |
| aminoacetic acid (glycine) | 2.4 |
| L-histidine monohydrochloride.$H_2O$ | 2.5 (1.9) |
| L-proline | 2.7 |
| L-serine | 4.9 |
| Total lipids | 80 g |
| medium-chain fatty acid triglyceride | 68.0 |
| soybean oil | 12.0 |
| Total vitamins | 100 g |
| phytonadione | 100 |
| Total emulsifiers | 7.6 g |
| purified soybean lecithin[1] | 1.2 |
| sucrose fatty acid ester[2] | 6.4 |
| Purified water | about 860 ml (pH 7.5) |

In the same manner as in Example 1, an aqueous solution of sucrose fatty acid ester and a mixture of a lipid and soybean lecithin were subjected to a first pre-emulsification with a homomixer. Then, amino acids were added and the solution was adjusted to a pH of 7.5 with a 8N potassium hydroxide aqueous solution. Water was added in an amount to bring the total amount to 1000 ml. The resulting solution was subjected to a second pre-emulsification and fine emulsification, filtered, placed into containers and sterilized, giving the nutritive emulsion of the invention.

Example 8

| Total amino acids (free) | 73 g (67) |
|---|---|
| L-isoleucine | 4.0 |
| L-leucine | 6.0 |
| L-lysine monohydrochloride | 7.4 (5.9) |
| L-methionine | 4.0 |
| L-phenylalanine | 5.0 |
| L-threonine | 5.0 |
| L-tryptophan | 1.4 |
| L-valine | 4.7 |
| L-alanine | 5.0 |
| L-arginine monohydrochloride | 8.0 (6.6) |
| monosodium L-aspartate.$H_2O$ | 3.3 (2.5) |
| monosodium L-glutamate.$H_2O$ | 8.5 (6.7) |
| aminoacetic acid (glycine) | 2.0 |
| L-histidine monohydrochloride $H_2O$ | 2.1 (1.6) |
| L-proline | 2.3 |
| L-serine | 4.3 |
| Total lipids | 80 g |
| medium-chain fatty acid triglyceride | 68.0 |
| soybean oil | 12.0 |
| Total emulsifiers | 7.6 g |
| purified soybean lecithin[1] | 1.2 |
| sucrose fatty acid ester[2] | 6.4 |
| Purified water | about 860 ml (pH 7.5) |

In the same manner as in Example 1, an aqueous solution of sucrose fatty acid ester and a mixture of a lipid and soybean lecithin were subjected to a first pre-emulsification with a homomixer. Then, amino acids were added and the solution was adjusted to a pH of 7.5 with a 8N potassium hydroxide aqueous solution. Water was added in an amount to bring the total amount to 1000 ml. The resulting solution was subjected to a second pre-emulsification and fine emulsification, filtered, placed into containers and sterilized, giving the nutritive emulsion of the invention.

16

## Example 9

| | |
|---|---|
| Total amino acids (free) | 55.7 g (52.2) |
| L-isoleucine | 6.0 |
| L-leucine | 9.1 |
| L-lysine monohydrochloride | 4.3 (3.4) |
| L-methionine | 2.3 |
| L-phenylalanine | 2.8 |
| L-threonine | 2.9 |
| L-tryptophan | 0.8 |
| L-valine | 7.1 |
| L-alanine | 2.9 |
| L-arginine monohydrochloride | 4.6 (3.8) |
| monosodium L-aspartate.$H_2O$ | 1.9 (1.5) |
| monosodium L-glutamate.$H_2O$ | 4.9 (3.8) |
| aminoacetic acid (glycine) | 1.2 |
| L-histidine monohydrochloride $H_2O$ | 1.2 (0.9) |
| L-proline | 1.3 |
| L-serine | 2.4 |
| Total lipids | 80 g |
| medium-chain fatty acid triglyceride | 68.0 |
| soybean oil | 12.0 |
| Total emulsifiers | 7.6 g |
| purified soybean lecithin[1] | 1.2 |
| sucrose fatty acid ester[2] | 6.4 |
| Purified water | about 860 ml (pH 7.5) |

In the same manner as in Example 1, an aqueous solution of sucrose fatty acid ester and a mixture of a lipid and soybean lecithin were subjected to a first pre-emulsification with a homomixer. Then, amino acids were added and the solution was adjusted to a pH of 7.5 with a 8N potassium hydroxide aqueous solution. Water was added in an amount to bring the total amount to 1000 ml. The resulting solution was subjected to a second pre-emulsification and fine emulsification, filtered, placed into containers and sterilized, giving the nutritive emulsion of the invention.

Example 10

| | |
|---|---|
| Total amino acids (free) | 48.2 g (44.8) |
| L-isoleucine | 4.0 |
| L-leucine | 6.0 |
| L-lysine mcnohydrochloride | 4.3 (3.4) |
| L-methionine | 2.3 |
| L-phenylalanine | 2.8 |
| L-threonine | 2.9 |
| L-tryptophan | 0.8 |
| L-valine | 4.7 |
| L-alanine | 2.9 |
| L-arginine monohydrochloride | 4.6 (3.8) |
| monosodium L-aspartate.$H_2O$ | 1.9 (1.5) |
| monosodium L-glutamate.$H_2O$ | 4.9 (3.8) |
| aminoacetic acid (glycine) | 1.2 |
| L-histidine monohydrochloride $H_2O$ | 1.2 (0.9) |
| L-proline | 1.3 |
| L-serine | 2.4 |
| Total lipids | 40 g |
| medium-chain fatty acid triglyceride | 34.0 |
| soybean oil | 6.0 |
| Total emulsifiers | 5.3 g |
| purified soybean lecithin[1] | 0.8 |
| sucrose fatty acid ester[2] | 4.5 |
| Purified water | about 860 ml (pH 7.5) |

In the same manner as in Examlple 1, an aqueous solution of sucrose fatty acid ester and a mixture of a lipid and soybean lecithin were subjected to a first pre-emulsification with a homomixer. Then, amino acids were added and the solution was adjusted to a pH of 7.5 with a 8N potassium hydroxide aqueous solution. Water was added in an amount to bring the total amount to 1000 ml. The resulting solution was subjected to a second pre-emulsification and fine emulsification, filtered, placed into containers and sterilized, giving the nutritive emulsion of the invention.

Example 11

| | |
|---|---|
| Total amino acids (free) | 97 g (90.1) |
| L-isoleucine | 8.0 |
| L-leucine | 12.1 |
| L-lysine monohydrochloride | 8.6 (6.9) |
| L-methionine | 4.6 |
| L-phenylalanine | 5.7 |
| L-threonine | 5.8 |
| L-tryptophan | 1.6 |
| L-valine | 9.4 |
| L-alanine | 5.8 |
| L-arginine monohydrochloride | 9.2 (7.6) |
| monosodium L-aspartate.$H_2O$ | 3.9 (3.0) |
| monosodium L-glutamate.$H_2O$ | 9.8 (7.7) |
| aminoacetic acid (glycine) | 2.4 |
| L-histidine monohydrochloride $H_2O$ | 2.5 (1.9) |
| L-proline | 2.7 |
| L-serine | 4.9 |
| Total lipids | 80 g |
| medium-chain fatty acid triglyceride | 68.0 |
| soybean oil | 12.0 |
| Total vitamins | 100 μg |
| phytonadione | 100 |
| Total minerals | 1.3 g |
| potassium hydroxide | 1.3 |
| Total emulsifiers | 7.6 g |
| purified soybean lecithin[1] | 1.2 |
| sucrose fatty acid ester[3] | 6.4 |
| Purified water | about 860 ml (pH 7.5) |

(Note)
3)"DK-F-160" (product of Daiichi Kogyo Seiyaku Co.,Ltd., HLB 15.0)

In the same manner as in Example 1, an aqueous solution of sucrose fatty acid ester and a mixture of a lipid and soybean lecithin were subjected to a first pre-emulsification with a homomixer. Then, amino acids were added and the solution was adjusted to a pH of 7.5 with a 8N potassium hydroxide aqueous solution. Water was added in an amount to bring the total amount to 1000 ml. The resulting solution was subjected to a second pre-emulsification and fine emulsification, filtered, placed into containers and sterilized, giving the nutritive emulsion of the invention.

Example 12

| | |
|---|---|
| Total amino acids (free) | 97 g (90.1) |
| L-isoleucine | 8.0 |

| | |
|---|---|
| L-leucine | 12.1 |
| L-lysine monohydrochloride | 8.6 (6.9) |
| L-methionine | 4.6 |
| L-phenylalanine | 5.7 |
| L-threonine | 5.8 |
| L-tryptophan | 1.6 |
| L-valine | 9.4 |
| L-alanine | 5.8 |
| L-arginine monohydrochloride | 9.2 (7.6) |
| monosodium L-aspartate.$H_2O$ | 3.9 (3.0) |
| monosodium L-glutamate.$H_2O$ | 9.8 (7.7) |
| aminoacetic acid (glycine) | 2.4 |
| L-histidine monohydrochloride $H_2O$ | 2.5 (1.9) |
| L-proline | 2.7 |
| L-serine | 4.9 |
| Total lipids | 80 g |
| medium-chain fatty acid triglyceride | 60.0 |
| soybean oil | 20.0 |
| Total vitamins | 100 µg |
| phytonadione | 100 |
| Total minerals | 1.3 g |
| potassium hydroxide | 1.3 |
| Total emulsifiers | 7.6 g |
| purified soybean lecithin[1] | 1.2 |
| sucrose fatty acid ester[2] | 6.4 |

| | |
|---|---|
| Purified water | about 860 ml (pH 7.5) |

20

In the same manner as in Example 1, an aqueous solution of sucrose fatty acid ester and a mixture of a lipid and soybean lecithin were subjected to a first pre-emulsification with a homomixer. Then, amino acids were added and the solution was adjusted to a pH of 7.5 with a 8N potassium hydroxide aqueous solution. Water was added in an amount to bring the total amount to 1000 ml. The resulting solution was subjected to a second pre-emulsification and fine emulsification, filtered, placed into containers and sterilized, giving the nutritive emulsion of the invention.

Example 13

| Total amino acids (free) | 104.4 g (97.5) |
|---|---|
| L-isoleucine | 11.9 |
| L-leucine | 18.1 |
| L-lysine monohydrochloride | 8.6 (6.9) |
| L-methionine | 4.6 |
| L-phenylalanine | 5.7 |
| L-threonine | 3.7 |
| L-tryptophan | 1.6 |
| L-valine | 9.0 |
| L-alanine | 5.8 |
| L-arginine monohydrochloride | 9.2 (7.6) |
| monosodium L-aspartate.$H_2O$ | 3.9 (3.0) |

21

| | |
|---|---|
| monosodium L-glutamate.$H_2O$ | 9.8 (7.7) |
| aminoacetic acid (glycine) | 2.4 |
| L-histidine monohydrochloride $H_2O$ | 2.5 (1.9) |
| L-proline | 2.7 |
| L-serine | 4.9 |
| Total lipids | 80 g |
| medium-chain fatty acid triglyceride | 64.0 |
| soybean oil | 16.0 |
| Total vitamins | 100 μg |
| phytonadione | 100 |
| Total minerals | 1.3 g |
| potassium hydroxide | 1.3 |
| Total emulsifiers | 7.6 g |
| purified soybean lecithin[1] | 1.2 |
| sucrose fatty acid ester[2] | 6.4 |
| Purified water | about 860 ml (pH 7.5) |

In the same manner as in Example 1, an aqueous solution of sucrose fatty acid ester and a mixture of a lipid and soybean lecithin were subjected to a first pre-emulsification with a homomixer. Then, amino acids were added and the solution was adjusted to a pH of 7.5 with a 8N potassium hydroxide aqueous solution. Water was added in an amount to bring the total amount to 1000 ml. The resulting solution was subjected to a second pre-emulsification and fine emulsification, filtered, placed into containers and sterilized, giving the nutritive emulsion of the invention.

Example 14

| Total amino acids + peptides (free) | 97 g (90.9) |
|---|---|
| L-isoleucine | 8.0 |
| L-leucine | 12.1 |
| L-lysine monohydrochloride | 8.6 (6.9) |
| L-methionine | 4.6 |
| L-alanyl-L-phenylalanine | 11.5 (12.3) |
| L-threonine | 5.8 |
| L-tryptophan | 1.6 |
| L-valine | 9.4 |
| L-arginine monohydrochloride | 9.2 (7.6) |
| monosodium L-aspartate.$H_2O$ | 3.9 (3.0) |
| monosodium L-glutamate.$H_2O$ | 9.8 (7.7) |
| aminoacetic acid (glycine) | 2.4 |
| L-histidine monohydrochloride $H_2O$ | 2.5 (1.9) |
| L-proline | 2.7 |
| L-serine | 4.9 |
| Total lipids | 80 g |
| medium-chain fatty acid triglyceride | 68.0 |
| soybean oil | 12.0 |
| Total vitamins | 100 μg |
| phytonadione | 100 |
| Total minerals | 1.3 g |
| potassium hydroxide | 1.3 |
| Total emulsifiers | 7.6 g |
| purified soybean lecithin[1] | 1.2 |
| sucrose fatty acid ester[2] | 6.4 |
| Purified water | about 860 ml (pH 7.5) |

In the same manner as in Example 1, an aqueous solution of sucrose fatty acid ester and a mixture of a lipid and soybean lecithin were subjected to a first pre-emulsification with a homomixer. Then, amino acids were added and the solution was adjusted to a pH of 7.5 with a 8N potassium hydroxide aqueous solution. Water was added in an amount to bring the total amount to 1000 ml. The resulting solution was subjected to a second pre-emulsification and fine emulsification, filtered, placed into containers and sterilized, giving the nutritive emulsion of the invention.

Example 15

| | |
|---|---|
| Total amino acids + peptides (free) | 104.0 g (97.7) |
| L-isoleucine | 8.9 |
| L-leucyl-L-valine | 20.1 (21.6) |
| L-lysine monohydrochloride | 9.6 (7.7) |
| L-methionine | 5.1 |
| L-phenylalanine | 6.3 |
| L-threonine | 6.4 |
| L-tryptophan | 1.8 |
| L-alanine | 6.4 |
| L-arginine monohydrochloride | 10.2 (8.4) |
| monosodium L-aspartate.$H_2O$ | 4.3 (3.3) |
| monosodium L-glutamate.$H_2O$ | 11.0 (8.6) |
| aminoacetic acid (glycine) | 2.7 |
| L-histidine monohydrochloride $H_2O$ | 2.8 (2.1) |
| L-proline | 3.0 |
| L-serine | 5.4 |
| Total lipids | 80.0 g |
| medium-chain fatty acid triglyceride | 68.0 |
| soybean oil | 12.0 |
| Total vitamins | 100 $\mu$g |
| phytonadione | 100 |
| Total emulsifiers | 7.6 g |
| purified soybean lecithin[1] | 1.2 |
| sucrose fatty acid ester[2] | 6.4 |
| Purified water | about 860 ml (pH 7.5) |

In the same manner as in Example 1, an aqueous solution of sucrose fatty acid ester and a mixture of a lipid and soybean lecithin were subjected to a first pre-emulsification with a homomixer. Then, amino acids were added and the solution was adjusted to a pH of 7.5 with a 8N potassium hydroxide aqueous solution. Water was added in an amount to bring the total amount to 1000 ml. The resulting solution was subjected to a second pre-emulsification and fine emulsification, filtered, placed into containers and sterilized, giving the nutritive emulsion of the invention.

Example 16

| | |
|---|---|
| Total amino acids + peptides (free) | 97 g (90.1) |
| L-isoleucine | 8.0 |
| L-leucine | 12.1 |
| L-lysine monohydrochloride | 8.6 (6.9) |
| L-methionine | 4.6 |
| L-phenylalanine | 5.7 |
| L-threonine | 5.8 |
| L-tryptophan | 1.6 |
| L-valine | 9.4 |
| glycyl-L-alanine | 8.2 (9.1) |
| L-arginine monohydrochloride | 9.2 (7.6) |
| monosodium L-aspartate.$H_2O$ | 3.9 (3.0) |
| monosodium L-glutamate.$H_2O$ | 9.8 (7.7) |
| L-histidine monohydrochloride $H_2O$ | 2.5 (1.9) |
| L-proline | 2.7 |
| L-serine | 4.9 |
| Total lipids | 80.0 g |
| medium-chain fatty acid triglyceride | 68.0 |
| soybean oil | 12.0 |
| Total emulsifiers | 7.0 g |
| purified soybean lecithin[1] | 1.1 |
| sucrose fatty acid ester[2] | 5.9 |
| Purified water | about 860 ml (pH 7.5) |

In the same manner as in Example 1, an aqueous solution of sucrose fatty acid ester and a mixture of a lipid and soybean lecithin were subjected to a first pre-emulsification with a homomixer. Then, amino acids were added and the solution was adjusted to a pH of 7.5 with a 8N potassium hydroxide aqueous solution. Water was added in an amount to bring the total amount to 1000 ml. The resulting solution was subjected to a second pre-emulsification and fine emulsification, filtered, placed into containers and sterilized, giving the nutritive emulsion of the invention.

Example 17

| Total amino acids + peptides (free) | 110.5 g (104.8) |
|---|---|
| L-isoleucine | 12.0 |
| L-leucyl-L-leucine | 16.9 (18.1) |
| L-lysine monohydrochloride | 8.6 (6.9) |
| L-methionine | 4.6 |
| L-phenylalanine | 5.7 |
| L-threonine | 5.8 |
| L-tryptophan | 1.6 |
| L-valine | 14.1 |
| L-alanine | 5.8 |
| L-arginine monohydrochloride | 9.2 (7.6) |
| monosodium L-aspartate.$H_2O$ | 3.9 (3.0) |
| monosodium L-glutamate.$H_2O$ | 9.8 (7.7) |
| aminoacetic acid (glycine) | 2.4 |
| L-histidine monohydrochloride $H_2O$ | 2.5 (1.9) |
| L-proline | 2.7 |
| L-serine | 4.9 |
| Total lipids | 80.0 g |
| medium-chain fatty acid triglyceride | 68.0 |
| soybean oil | 12.0 |
| Total vitamins | 100 μg |
| phytonadione | 100 |
| Total emulsifiers | 7.6 g |
| purified soybean lecithin[1] | 1.2 |
| sucrose fatty acid ester[2] | 6.4 |
| Purified water | about 860 ml (pH 7.5) |

In the same manner as in Example 1, an aqueous solution of sucrose fatty acid ester and a mixture of a lipid and soybean lecithin were subjected to a first pre-emulsification with a homomixer. Then, amino acids were added and the solution was adjusted to a pH of 7.5 with a 8N potassium hydroxide aqueous solution. Water was added in an amount to bring the total amount to 1000 ml. The resulting solution was subjected to a second pre-emulsification and fine emulsification, filtered, placed into containers and sterilized, giving the nutritive emulsion of the invention.

Example 18

| Total amino acids + peptides (free) | 55.7 g (52.4) |
|---|---|
| L-isoleucine | 6.0 |
| L-leucine | 9.1 |
| L-lysine monohydrochloride | 4.3 (3.4) |
| L-methionine | 2.3 |
| L-phenylalanine | 2.8 |
| L-threonine | 2.9 |
| L-tryptophyl-glycyl-glycine | 2.0 (2.2) |
| L-valine | 7.1 |
| L-alanine | 2.9 |
| L-arginine monohydrochloride | 4.5 (3.8) |
| monosodium L-aspartate.$H_2O$ | 1.9 (1.5) |
| monosodium L-glutamate.$H_2O$ | 4.9 (3.8) |
| L-histidine monohydrochloride $H_2O$ | 1.2 (0.9) |
| L-proline | 1.3 |
| L-serine | 2.4 |
| Total lipids | 80 g |
| medium-chain fatty acid triglyceride | 68.0 |
| soybean oil | 12.0 |
| Total emulsifiers | 7.6 g |
| purified soybean lecithin[1] | 1.2 |
| sucrose fatty acid ester[2] | 6.4 |
| Purified water | about 860 ml (pH 7.5) |

In the same manner as in Example 1, an aqueous solution of sucrose fatty acid ester and a mixture of a lipid and soybean lecithin were subjected to a first pre-emulsification with a homomixer. Then, amino acids were added and the solution was adjusted to a pH of 7.5 with a 8N potassium hydroxide aqueous solution. Water was added in an amount to bring the total amount to 1000 ml. The resulting solution was subjected to a second pre-emulsification and fine emulsification, filtered, placed into containers and sterilized, giving the nutritive emulsion of the invention.

Example 19

| | |
|---|---|
| Total amino acids + peptides (free) | 97 g (90.5) |
| L-isoleucine | 8.0 |
| L-leucine | 12.1 |
| L-lysine monohydrochloride | 8.6 (6.9) |
| L-methionine | 4.6 |
| L-phenylalanine | 5.7 |
| L-threonine | 5.8 |
| L-tryptophan | 1.6 |
| L-valine | 9.4 |
| L-alanine | 5.8 |
| L-arginine monohydrochloride | 9.2 (7.6) |
| monosodium L-aspartate.$H_2O$ | 3.9 (3.0) |
| monosodium L-glutamate.$H_2O$ | 9.8 (7.7) |
| glycyl-glycyl-glycyl-glycine | 2.4 (2.8) |
| L-histidine monohydrochloride $H_2O$ | 2.5 (1.9) |
| L-proline | 2.7 |
| L-serine | 4.9 |
| Total lipids | 80 g |
| medium-chain fatty acid triglyceride | 68.0 |
| soybean oil | 12.0 |
| Total vitamins | 100 μg |
| phytonadione | 100 |
| Total minerals | 1.3 g |
| potassium hydroxide | 1.3 |
| Total emulsifiers | 7.6 g |
| purified soybean lecithin[1] | 1.2 |
| sucrose fatty acid ester[3] | 6.4 |
| Purified water | about 860 ml (pH 7.5) |

In the same manner as in Example 1, an aqueous solution of sucrose fatty acid ester and a mixture of a lipid and soybean lecithin were subjected to a first pre-emulsification with a homomixer. Then, amino acids were added and the solution was adjusted to a pH of 7.5 with a 8N potassium hydroxide aqueous solution. Water was added in an amount to bring the total amount to 1000 ml. The resulting solution was subjected to a second pre-emulsification and fine emulsification, filtered, placed into containers and sterilized, giving the nutritive emulsion of the invention.

Example 20

| | |
|---|---|
| Total amino acids (free) | 104.4 g (97.5) |
| L-isoleucine | 11.9 |
| L-leucine | 18.1 |
| L-lysine monohydrochloride | 8.6 (6.9) |
| L-methionine | 4.6 |
| L-phenylalanine | 5.7 |
| L-threonine | 3.7 |
| L-tryptophan | 1.6 |
| L-valine | 9.0 |
| L-alanine | 5.8 |
| L-arginine monohydrochloride | 9.2 (7.6) |
| monosodium L-aspartate.$H_2O$ | 3.9 (3.0) |
| monosodium L-glutamate.$H_2O$ | 9.8 (7.7) |
| aminoacetic acid (glycine) | 2.4 |
| L-histidine monohydrochloride $H_2O$ | 2.5 (1.9) |
| L-proline | 2.7 |
| L-serine | 4.9 |
| Total lipids | 80.0 g |
| medium-chain fatty acid triglyceride | 64.0 |
| soybean oil | 16.0 |
| Total vitamins | 100 $\mu$g |
| phytonadione | 100 |
| Total minerals | 1.3 g |
| potassium hydroxide | 1.3 |
| Total emulsifiers | 7.6 g |
| purified soybean lecithin[1] | 1.2 |
| sucrose fatty acid ester[2] | 6.4 |
| Total dietary fibers | 8.0 g |
| polydextrose | 8.0 |
| Purified water | about 860 ml (pH 7.5) |

In the same manner as in Example 1, an aqueous solution of sucrose fatty acid ester and a mixture of a lipid, soybean lecithin and dietary fiber were subjected to a first pre-emulsification with a homomixer. Then, amino acids were added and the solution was adjusted to a pH of 7.5 with a 8N potassium hydroxide aqueous solution. Water was added in an amount to bring the total amount to 1000 ml. The resulting solution was subjected to a second pre-emulsification and fine emulsification, filtered, placed into containers and sterilized, giving the nutritive emulsion of the invention.

Example 21

| | |
|---|---|
| Total amino acids (free) | 98.1 g (92.9) |
| L-isoleucine | 9.4 |
| L-leucine | 14.6 |
| L-lysine monohydrochloride | 16.9 (13.5) |
| L-methionine | 14.6 |
| L-phenylalanine | 14.6 |
| L-threonine | 6.7 |
| L-tryptophan | 3.3 |
| L-valine | 10.7 |
| L-histidine monohydrochloride $H_2O$ | 7.3 (5.5) |
| Total lipids | 80.0 g |
| medium-chain fatty acid triglyceride | 68.0 |
| soybean oil | 12.0 |
| Total vitamins | 100 $\mu$g |
| phytonadione | 100 |
| Total emulsifiers | 8.2 g |
| sucrose fatty acid ester[2) | 8.2 |
| Purified water | about 860 ml (pH 7.5) |

In the same manner as in Example 1, an aqueous solution of sucrose fatty acid ester and a mixture of a lipid and soybean lecithin were subjected to a first pre-emulsification with a homomixer. Then, amino acids were added and the solution was adjusted to a pH of 7.5 with a 8N potassium hydroxide aqueous solution. Water was added in an amount to bring the total amount to 1000 ml. The resulting solution was subjected to a second pre-emulsification and fine emulsification, filtered, placed into containers and sterilized, giving the nutritive emulsion of the invention.

Example 22

| | |
|---|---|
| Total amino acids (free) | 97 g (90.1) |
| L-isoleucine | 8.0 |
| L-leucine | 12.1 |
| L-lysine monohydrochloride | 8.6 (6.9) |
| L-methionine | 4.6 |
| L-phenylalanine | 5.7 |
| L-threonine | 5.8 |
| L-tryptophan | 1.6 |
| L-valine | 9.4 |
| L-alanine | 5.8 |
| L-arginine monohydrochloride | 9.2 (7.6) |
| monosodium L-aspartate.$H_2O$ | 3.9 (3.0) |
| monosodium L-glutamate.$H_2O$ | 9.8 (7.7) |
| aminoacetic acid (glycine) | 2.4 |
| L-histidine monohydrochloride $H_2O$ | 2.5 (1.9) |
| L-proline | 2.7 |
| L-serine | 4.9 |
| Total lipids | 80.0 g |
| medium-chain fatty acid triglyceride | 68.0 |
| soybean oil | 12.0 |
| Total emulsifiers | 18.6 g |
| purified soybean lecithin[1] | 3.6 |
| glycerine fatty acid ester[4] | 15.0 |
| Purified water | about 860 ml (pH 7.5) |

(Note)
4)"Decaglyn 1-M" (product of Nikko Chemicals, HLB 14)

In the same manner as in Example 1, an aqueous solution of glycerine fatty acid ester and a mixture of a lipid and soybean lecithin were subjected to a first pre-emulsification with a homomixer. Then, amino acids were added and the solution was adjusted to a pH of 7.5 with a 8N potassium hydroxide aqueous solution. Water was added in an amount to bring the total amount to 1000 ml. The resulting solution was subjected to a second pre-emulsification and fine emulsification, filtered, placed into containers and sterilized, giving the nutritive emulsion of the invention.

The nutritive emulsions each having pH 6.9 and pH 7.9 were prepared in the same manner as above with the exception of that the amount of 8N potassium hydroxide aqueous solution was changed.

31

Example 23

| | |
|---|---|
| Total amino acids (free) | 97 g (90.1) |
| L-isoleucine | 8.0 |
| L-leucine | 12.1 |
| L-lysine monohydrochloride | 8.6 (6.9) |
| L-methionine | 4.6 |
| L-phenylalanine | 5.7 |
| L-threonine | 5.8 |
| L-tryptophan | 1.6 |
| L-valine | 9.4 |
| L-alanine | 5.8 |
| L-arginine monohydrochloride | 9.2 (7.6) |
| monosodium L-aspartate.$H_2O$ | 3.9 (3.0) |
| monosodium L-glutamate.$H_2O$ | 9.8 (7.7) |
| aminoacetic acid (glycine) | 2.4 |
| L-histidine monohydrochloride $H_2O$ | 2.5 (1.9) |
| L-proline | 2.7 |
| L-serine | 4.9 |
| Total lipids | 104.0 g |
| medium-chain fatty acid triglyceride | 88.4 |
| soybean oil | 15.6 |
| Total emulsifiers | 19.2 g |
| glycerine fatty acid ester[4] | 19.2 |
| Purified water | about 860 ml (pH 7.5) |

In the same manner as in Example 1, an aqueous solution of glycerine fatty acid ester and a mixture of a lipid and soybean lecithin were subjected to a first pre-emulsification with a homomixer. Then, amino acids were added and the solution was adjusted to a pH of 7.5 with a 8N potassium hydroxide aqueous solution. Water was added in an amount to bring the total amount to 1000 ml. The resulting solution was subjected to a second pre-emulsification and fine emulsification, filtered, placed into containers and sterilized, giving the nutritive emulsion of the invention.

Example 24

| | |
|---|---|
| Total amino acids + peptides (free) | 104.0 g (97.7) |
| L-isoleucine | 8.9 |
| L-leucyl-L-valine | 20.1 (21.6) |
| L-lysine monohydrochloride | 9.6 (7.7) |
| L-methionine | 5.1 |
| L-phenylalanine | 6.3 |
| L-threonine | 6.4 |
| L-tryptophan | 1.8 |
| L-alanine | 6.4 |
| L-arginine monohydrochloride | 10.2 (8.4) |
| monosodium L-aspartate.$H_2O$ | 4.3 (3.3) |
| monosodium L-glutamate.$H_2O$ | 11.0 (8.6) |
| aminoacetic acid (glycine) | 2.7 |
| L-histidine monohydrochloride $H_2O$ | 2.8 (2.1) |
| L-proline | 3.0 |
| L-serine | 5.4 |
| Total lipids | 80.0 g |
| medium-chain fatty acid triglyceride | 68.0 |
| soybean oil | 12.0 |
| Total vitamins | 100 $\mu$g |
| phytonadione | 100 |
| Total emulsifiers | 8.2 g |
| sucrose fatty acid ester[2] | 8.2 |
| Purified water | about 860 ml (pH 7.5) |

In the sane manner as in Example 1, an aqueous solution of sucrose fatty acid ester and a mixture of a lipid and soybean lecithin were subjected to a first pre-emulsification with a homomixer. Then, amino acids were added and the solution was adjusted to a pH of 7.5 with a 8N potassium hydroxide aqueous solution. Water was added in an amount to bring the total amount to 1000 ml. The resulting solution was subjected to a second pre-emulsification and fine emulsification, filtered, placed into containers and sterilized, giving the nutritive emulsion of the invention.

Example 25

| | |
|---|---|
| Total amino acids + peptide (free) | 110.5 g (104.8) |
| L-isoleucine | 12.0 |
| L-leucyl-L-leucine | 16.9 (18.1) |
| L-lysine monohydrochloride | 8.6 (6.9) |
| L-methionine | 4.6 |
| L-phenylalanine | 5.7 |
| L-threonine | 5.8 |
| L-tryptophan | 1.6 |
| L-valine | 14.1 |
| L-alanine | 5.8 |
| L-arginine monohydrochloride | 9.2 (7.6) |
| monosodium L-aspartate.$H_2O$ | 3.9 (3.0) |
| monosodium L-glutamate.$H_2O$ | 9.8 (7.7) |
| aminoacetic acid (glycine) | 2.4 |
| L-histidine monohydrochloride $H_2O$ | 2.5 (1.9) |
| L-proline | 2.7 |
| L-serine | 4.9 |
| Total lipids | 80.0 g |
| medium-chain fatty acid triglyceride | 68.0 |
| soybean oil | 12.0 |
| Total vitamins | 100 μg |
| phytonadione | 100 |
| Total emulsifiers | 18.6 g |
| purified soybean lecithin[1] | 3.6 |
| glycerine fatty acid ester[4] | 15.0 |
| Purified water | about 860 ml (pH 7.5) |

In the same manner as in Example 1, an aqueous solution of glycerine fatty acid ester and a mixture of a lipid and soybean lecithin were subjected to a first pre-emulsification with a homomixer. Then, amino acids were added and the solution was adjusted to a pH of 7.5 with a 8N potassium hydroxide aqueous solution. Water was added in an amount to bring the total amount to 1000 ml. The resulting solution was subjected to a second pre-emulsification and fine emulsification, filtered, placed into containers and sterilized, giving the nutritive emulsion of the invention.

Example 26

| | |
|---|---|
| Total amino acids + peptides (free) | 97 g (90.5) |
| L-isoleucine | 8.0 |
| L-leucine | 12.1 |
| L-lysine monohydrochloride | 8.6 (6.9) |
| L-methionine | 4.6 |
| L-phenylalanine | 5.7 |
| L-threonine | 5.8 |
| L-tryptophan | 1.6 |
| L-valine | 9.4 |
| L-alanine | 5.8 |
| L-arginine monohydrochloride | 9.2 (7.6) |
| monosodium L-aspartate.$H_2O$ | 3.9 (3.0) |
| monosodium L-glutamate.$H_2O$ | 9.8 (7.7) |
| glycyl-glycyl-glycyl-glycine | 2.4 (2.8) |
| L-histidine monohydrochloride $H_2O$ | 2.5 (1.9) |
| L-proline | 2.7 |
| L-serine | 4.9 |
| Total lipids | 80 g |
| medium-chain fatty acid triglyceride | 68.0 |
| soybean oil | 12.0 |
| Total vitamins | 100 $\mu$g |
| phytonadione | 100 |
| Total minerals | 1.3 g |
| potassium hydroxide | 1.3 |
| Total emulsifiers | 19.2 g |
| glycerine fatty acid ester[4] | 19.2 |
| Purified water | about 860 ml (pH 7.5) |

In the same manner as in Example 1, an aqueous solution of glycerine fatty acid ester and a mixture of a lipid and soybean lecithin were subjected to a first pre-emulsification with a homomixer. Then, amino acids were added and the solution was adjusted to a pH of 7.5 with a 8N potassium hydroxide aqueous solution. Water was added in an amount to bring the total amount to 1000 ml. The resulting solution was subjected to a second pre-emulsification and fine emulsification, filtered, placed into containers and sterilized, giving the nutritive emulsion of the invention.

Example 27

| | |
|---|---|
| Total amino acids (free) | 73 g (67) |
| L-isoleucine | 4.0 |
| L-leucine | 6.0 |
| L-lysine monohydrochloride | 7.4 (5.9) |
| L-methionine | 4.0 |
| L-phenylalanine | 5.0 |
| L-threonine | 5.0 |
| L-tryptophan | 1.4 |
| L-valine | 4.7 |
| L-alanine | 5.0 |
| L-arginine monohydrochloride | 8.0 (6.6) |
| monosodium L-aspartate.$H_2O$ | 3.3 (2.5) |
| monosodium L-glutamate.$H_2O$ | 8.5 (6.7) |
| aminoacetic acid (glycine) | 2.0 |
| L-histidine monohydrochloride $H_2O$ | 2.1 (1.6) |
| L-proline | 2.3 |
| L-serine | 4.3 |
| Total lipids | 80.0 g |
| medium-chain fatty acid triglyceride | 68.0 |
| soybean oil | 12.0 |
| Total emulsifiers | 17.0 g |
| purified soybean lecithin[1] | 5.0 |
| polyoxyethylene glycerin fatty acid ester[5] | 12.0 |
| Purified water | about 860 ml (pH 7.5) |

(Note)
5)"TMGS-15"(product of Nikko Chemicals, HLB 13.5)

In the same manner as in Example 1, an aqueous solution of polyoxyethylene glycerin fatty acid ester and a mixture of a lipid and soybean lecithin were subjected to a first pre-emulsification with a homomixer. Then, amino acids were added and the solution was adjusted to a pH of 7.5 with a 8N potassium hydroxide aqueous solution. Water was added in an amount to bring the total amount to 1000 ml. The resulting solution was subjected to a second pre-emulsification and fine emulsification, filtered, placed into containers and sterilized, giving the nutritive emulsion of the invention.

Example 28

| Total amino acids (free) | 48.2 g (44.8) |
|---|---|
| L-isoleucine | 4.0 |
| L-leucine | 6.0 |
| L-lysine monohydrochloride | 4.3 (3.4) |
| L-methionine | 2.3 |
| L-phenylalanine | 2.8 |
| L-threonine | 2.9 |
| L-tryptophan | 0.8 |
| L-valine | 4.7 |
| L-alanine | 2.9 |
| L-arginine monohydrochloride | 4.6 (3.8) |
| monosodium L-aspartate.$H_2O$ | 1.9 (1.5) |
| monosodium L-glutamate.$H_2O$ | 4.9 (3.8) |
| aminoacetic acid (glycine) | 1.2 |
| L-histidine monohydrochloride $H_2O$ | 1.2 (0.9) |
| L-proline | 1.3 |
| L-serine | 2.4 |
| Total lipids | 40.0 g |
| medium-chain fatty acid triglyceride | 34.0 |
| soybean oil | 6.0 |
| Total emulsifiers | 15.0 g |
| purified soybean lecithin[1] | 5.0 |
| polyoxyethylene sorbitan fatty acid ester[6] | 10.0 |
| Purified water | about 860 ml (pH 7.5) |

(Note)
6)"TO-10"(product of Nikko Chemicals, HLB 15)

In the same manner as in Example 1, an aqueous solution of polyoxyethylene sorbitan fatty acid ester and a mixture of a lipid and soybean lecithin were subjected to a first pre-emulsification with a homomixer. Then, amino acids were added and the solution was adjusted to a pH of 7.5 with a 8N potassium hydroxide aqueous solution. Water was added in an amount to bring the total amount to 1000 ml. The resulting solution was subjected to a second pre-emulsification and fine emulsification, filtered, placed into containers and sterilized, giving the nutritive emulsion of the invention.

The nutritive emulsions of the invention prepared in the Examples were tested for emulsion stability. The test was conducted as follows.

Emulsion stability test

Each of emulsion samples obtained in the Examples was centrifuged at 3000 rpm far 10 minutes before or after sterilization, placed into test tubes and left to stand. Then the separation of liquids in the samples was observed with the unaided eye and the distribution of the emulsion particle size was determined.

Table 4 shows the test results of the samples of Examples 1 and 5 centrifuged before sterilization and Table 5 the test results of the samples centrifuged after sterilization. Table 6 shows the test results of three kinds of samples obtained in Example 4 with different pH values and centrifuged after sterilization.

Table 4

| (Before sterilizaiton) | | |
|---|---|---|
| Sample | Example 1 | Example 5 |
| Size distribution ($\mu$m) | | |
| 0.15 | 10 | 10 |
| 0.20 | 13 | 13 |
| 0.30 | 11 | 12 |
| 0.39 | 21 | 20 |
| 0.55 | 25 | 19 |
| 0.80 | 17 | 18 |
| 1.30 | 0 | 4 |
| 2.21 | 0 | 0 |
| 3.13 | 0 | 0 |
| 4.43 | 0 | 0 |
| Average particle size | 0.44 | 0.47 |
| Appearance after centrifugation | not separated | not separated |

Table 5

| (After sterilizaiton) | | |
|---|---|---|
| Sample | Example 1 | Example 5 |
| Size distribution ($\mu$m) | | |
| 0.15 | 9 | 6 |
| 0.20 | 12 | 13 |
| 0.30 | 13 | 9 |
| 0.39 | 22 | 21 |
| 0.55 | 23 | 30 |
| 0.80 | 17 | 24 |
| 1.30 | 0 | 0 |
| 2.21 | 0 | 0 |
| 3.13 | 0 | 0 |
| 4.43 | 0 | 0 |
| Average particle size | 0.44 | 0.49 |
| Appearance after centrifugation | not separated | not separated |

Table 6

| (After sterilizaiton) | | | |
|---|---|---|---|
| Sample | Nutritive emulsions of Example 4 | | |
| pH | 6.9 | 7.5 | 7.9 |
| Size distribution (μm) | | | |
| 0.15 | 7 | 5 | 4 |
| 0.20 | 9 | 7 | 5 |
| 0.30 | 12 | 9 | 6 |
| 0.39 | 22 | 19 | 17 |
| 0.55 | 25 | 29 | 31 |
| 0.80 | 20 | 24 | 27 |
| 1.30 | 2 | 3 | 6 |
| 2.21 | 0 | 0 | 0 |
| Average particle size | 0.48 | 0.53 | 0.58 |
| Appearance after centrifugation | not separated | not separated | not separated |

As shown in Tables 4, 5 and 6, the nutritive emulsions of the present invention all exhibit excellent emulsion stability after centrifugation and have the features of being satisfactory in appearance and in size distribution.

The comprehensive nutritive agents containing the nutritive emulsion of the invention are described below in detail with reference to Reference Examples.

Reference Example 1

A powder composition was prepared for addition to the nutritive emulsion of the invention. The powder comprised the following sugar, vitamins and minerals in the amounts listed below:

| Total sugars | 330 g |
|---|---|
| Maltose | 330 |
| Total vitamins | 283.976 mg |
| Retinol palmitate granule | 16.7 mg(5000IU) |
| Bisbentiamine | 2.2 mg |
| Riboflavin | 1.7 mg |
| Pyridoxine hydrochloride | 2.5 mg |
| Cyanocobalamin | 6 $\mu$g |
| Sodium ascorbate | 65.5 mg |
| Cholecalciferol granule | 1.67 mg(500IU) |
| Tocopherol acetate granule | 40.0 mg(10.0 mg) |
| Nicotinamide | 20.0 mg |
| Folic acid | 400 $\mu$g |
| Calcium pantothenate | 11.0 mg |
| Biotin | 300 $\mu$g |
| Choline bitartrate | 122.0 mg |
| Total minerals | 12.824 g |
| Calcium glycerophosphate | 5.23 g |
| Dipotassium monohydrogenphosphate | 1.28 g |
| Magnesium sulfate.7$H_2$O | 4.056 g |
| Sodium ferrous citrate | 0.164 g |
| Manganese sulfate.5$H_2$O | 11 mg |
| Copper sulfate.5$H_2$O | 8 mg |
| Zinc sulfate.7$H_2$O | 66 mg |
| Potassium iodide | 196.2 $\mu$g |
| Potassium sorbate | 2.0 g |
| Others (pH adjusting agent) | 3.6 g |
| Citric anhydride | 3.6 g |
| TOTAL | 346.7 g |

The above components were each weighed, pulverized and mixed together to give a powder composition.

The powder obtained was mixed with 1000 ml of each of the nutritive emulsions of the invention respectively prepared in Examples 1 and 18, affording a comprehensive nutritive agent (total amount: 1200 ml).

The powder was rapidly dispersed into the emulsion after addition. The obtained comprehensive nutritive agent was allowed to stand for 24 hours and the appearance of the agent was observed with no change found. When the appearance of the agent was observed after centrifugation as done in the emulsion stability test, the agent showed no separation of liquids, indicating a satisfactory emulsion stability.

Further, the obtained comprehensive nutritive agent was adjusted to a pH of about 5.2 with a citric anhydride. This pH adjustment rendered the vitamins (sodium ascorbate, etc.) free of instability. The pH adjustment, coupled with the potent bacteriostatic effect of potassium sorbate (serving also as a preservative) added, results in sufficient inhibition of growth of bacteria, whereby it was confirmed that the comprehensive nutritive agent is satisfactorily usable as a high-calory nutritive agent (2400 Kcal/day).

Reference Example 2

A powder composition was prepared in the same manner as in Reference Example 1 except that 2.0 g of potassium sorbate used as the mineral was replaced by 0.5 g of potassium sorbate and 0.7 g of potassium chloride and that 3.5 g of citric anhydride was used.

The obtained powder was added to 1000 ml of each of the nutritive emulsions of the invention prepared in Examples 1 and 18, giving a comprehensive nutritive agent (total amount; 1200 ml).

The above powder was rapidly dispersed in the emulsion after addition. The resulting comprehensive nutritive agent had a satisfactory emulsion stability.

The comprehensive nutritive agents obtained in Reference Example 1 (i.e. the agents each containing the nutritive emulsions respectively of Examples 1 and 18) was administered to hepatectomized rats which had a post-operative stress as well as reduced liver-function, and then the rats were checked for nutritive effect as described below in detail in the following Test Examples.

Test Example 1

Animals used in the test were male Wister-rats weighing about 230 g and fasted before the test for 24 hours after 1 week of preliminary feeding. The rats were 70% hepatectomized under anesthesia with ether by the Higgins-Anderson method (Higgins, G.M. and Anderson, R.M. Arch. Pathol., 12, 186 (1931)). At the same time, gastrostomy was done by the Witzel method (Witzel O., Zbl. Chir., 18, 601 (1891)), and the test nutritive agent was continuously administered for 24 hours through a silicone tube inserted from the vestibule of gaster to dodecadactylon.

The rats were divided into two groups of A and B. The A group was given a mixture of the nutritive emulsion of the invention and the powder composition (comprehensive nutrititve agent formulated to give 1 Kcal/ml), and the B group was given a commercially available nutritive agent (product of Morishita Co., Ltd. and Ajinomoto Co., Ltd., tradename "Elental", formulated to give 1 Kcal/ml). The sham-hepatectomized groups each corresponding to A and B groups were used as controls.

The nutritive agent was given the rats at a dose corresponding to about 75 Kcal/kg/day on the 1st disease day after operation. The dose was adjusted thereafter to increase the calory stepwise every day to a constant value of about 300 Kcal/kg/day on the 4th disease day.

Seven days after the beginning of the test, blood samples were collected from rats' abdominal aorta and animals were sacrificed. The following nutritive indexes were determined for consideration.

The results of consideration described below are common with the comprehensive nutritive agents each containing the nutritive emulsions respectively of Example 1 and Example 18.

1. Increase of body weight

There was no significant difference between A and B groups on each disease day. The body weight was recovered on the 7th disease day to approximately the value shown before the pre-operative days.

2. Liver weight

A and B groups both showed a high recovery rate of liver weight of about 75%. No significant difference was found between the two groups. The recovery rate of liver weight is given by the following equation:

$$\text{Recovery rate} = \frac{\text{liver wt. of test group/100g body wt.}}{\text{liver wt. of control group/100g body wt.}}$$

3. Urinary 3-methylhistidine/creatinine ratio

On the 1st disease day, the A and B groups both showed a high ratio but the ratio wan decreased with time to about 0.04 on and after the 4th disease day. There was no significant difference between the two groups.

4. Nitrogen balance (change of nitrogen)

A minus tendency was slightly stronger in A group than in B group on the 1st disease day. However, there was no significant difference between the two groups. The value changed to plus on the 3rd disease day in both groups.

5. Biochemical test of plasma

There was no significant difference between A and B gorups.

41

6. Plasma amino acid pattern

A pronouncedly greater increase of branched-chain amino acid (Leu, Ile, Val) was seen in A group than in B group, and a decrease of Tyr was observed in A group, resulting in an increase of Fischer's ratio.

7. Plasma fatty acid pattern

Group B showed decreases of $C18:2\omega6$ (linoleic acid), $C18:3\omega3$ (linolenic acid) and $C20:4\omega6$ (arachidonic acid) and increases of $C16:1\omega7$ (palmitoleic acid) and $C20:3\omega9$, and finding of essential fatty acid deficiency.

On the other hand, A group indicated a remarkable alleviation of essential fatty acid deficiency, displaying a satisfactory plasma fatty acid pattern.

The above results reveal that the comprehensive nutritive agent comprising the nutritive emulsion of the invention and the powder composition can exhibit an excellent nutritive effect when administered.

**Claims**

1. A nutritive emulsion containing, as an essential component, an amino acid preparation, a lipid, water, and an emulsifier, which is characterized in that the emulsion comprises 4 to 15% by weight of the amino acid preparation having the following amino acid composition in which the amount of each amino acid is expressed in percentage by weight, 3 to 12% by weight of a mixture of medium-chain fatty acid triglyceride (MCT) and long-chain fatty acid triglyceride (LCT) as the lipid, 71.5 to 93% by weight of water, and 0.3 to 3.0% by weight of the emulsifier having an HLB of at least 13 and selected from the group consisting of sucrose fatty ester, glycerin fatty acid ester, polyoxyethylene sorbitan fatty acid ester and an emulsifier mixture of said emulsifier and soybean lecithin and in that the emulsifier has a pH of 6.5 to 8.5 :

| Amino acid | Amount (wt%) |
|---|---|
| L-isoleucine | 4.5-13.4 |
| L-leucine | 6.7-20.1 |
| L-lysine | 3.8-15.2 |
| L-methionine | 3.0-16.4 |
| L-phenylalamine | 3.1-16.4 |
| L-threonine | 3.2- 9.6 |
| L-tryptophan | 0.9- 3.7 |
| L-valine | 5.2-15.6 |
| L-alanine | 0 - 9.6 |
| L-arginine | 0 -12.6 |
| L-aspartic acid | 0 -12.2 |
| L-glutamic acid | 0 -12.9 |
| L-glutamine | 0 -16.2 |
| Aminoacetic acid (glycine) | 0 - 4.1 |
| L-histidine | 0 - 6.2 |
| L-proline | 0 - 5.3 |
| L-serine | 0 - 9.7 |
| L-tyrosine | 0 - 0.9 |

2. A nutritive emulsion according to claim 1 wherein the amino acid preparation has the following amino acid composition in which the amount of each amino acid is expressed in percentage by weight and calculated as free amino acids based on the total amino acids.

| Amino acid | Amount (wt%) |
|---|---|
| L-isoleucine | 4.5-13.4 |
| L-leucine | 6.7-20.1 |
| L-lysine | 3.8-11.6 |
| L-methionine | 3.3- 7.7 |
| L-phenylalanine | 3.1- 9.5 |
| L-threonine | 3.2- 9.6 |
| L-tryptophan | 0.9- 2.7 |
| L-valine | 5.2-15.6 |
| L-alanine | 3.2- 9.6 |
| L-arginine | 0.9- 2.7 |
| L-aspartic acid | 1.6- 5.0 |
| L-glutamic acid | 4.3-12.9 |
| Aminoacetic acid (glycine) | 1.3- 4.1 |
| L-histidine | 1.0- 3.2 |
| L-proline | 1.5- 4.5 |
| L-serine | 2.7- 8.1 |

3. A nutritive emulsion according to claim 1 wherein the amino acid preparation has the following amino acid composition in which the amount of each amino acid is expressed in percentage by weight and calculated as free amino acids based on the total amino acids.

| Amino acid | Amount (wt%) |
|---|---|
| L-isoleucine | 8.0-13.4 |
| L-leucine | 12.5-20.1 |
| L-lysine | 6.9- 8.5 |
| L-methionine | 4.6- 5.6 |
| L-phenylalanine | 5.6- 6.9 |
| L-threonine | 3.2- 7.0 |
| L-tryptophan | 1.6- 2.0 |
| L-valine | 5.2-11.4 |
| L-alanine | 5.8- 7.0 |
| L-arginine | 7.6- 9.2 |
| L-aspartic acid | 3.0- 3.6 |
| L-glutamic acid | 7.7- 9.5 |
| Aminoacetic acid (glycine) | 2.4- 3.0 |
| L-histidine | 1.9- 2.3 |
| L-proline | 2.7- 3.3 |
| L-serine | 4.9- 5.9 |

4. A nutritive emulsion according to any one of claims 1 to 3 wherein part of amino acids constituting said amino acid preparation is used in the form of di- to penta-peptides.

5. A nutritive emulsion according to any one of claims 1 to 4 wherein the emulsifier is a mixture of sucrose fatty acid ester and/or glycerin fatty acid ester having an HLB of not less than 13 and soybean lecithin, the ratio (wt%) of sucrose fatty acid ester and/or glycerin fatty acid ester to soybean lecithin is 75-90 : 10-25.

6. A nutritive emulsion according to any one of claims 1 to 5 wherein the lipid is a mixture of medium-chain fatty acid triglyceride (MCT) and long-chain fatty acid triglyceride (LCT) and the ratio (wt%) of the former to the latter is 50-90 : 10-50.

7. A process for preparing a nutritive emulsion, according to any one of Claims 1 to 6, characterized in that the process comprises the steps of adding a lipid or, when required, a mixture of a lipid and soybean lecithin to a mixture of water and an emulsifier, the emulsifier having an HLB of at least 13 and being selected from the group consisting of sucrose fatty acid ester, glycerin fatty acid ester, polyoxyethylene glycerin fatty acid ester and polyoxyethylene sorbitan fatty acid ester; subjecting the mixture to pre-emulsification; adding an aqueous solution of an amino acid preparation to the coarse emulsified liquid; and fully emulsifying the mixture.

**Patentansprüche**

1. Nährmittelemulsion, enthaltend als wesentliche Komponente ein Aminosäurepräparat, ein Lipid, Wasser und einen Emulgator, dadurch **gekennzeichnet,** daß die Emulsion 4 bis 15 Gew.-% des Aminosäurepräparats mit der folgenden Aminosäurezusammensetzung, worin die Menge jeder Aminosäure als Gewichtsprozent ausgedrückt ist, 3 bis 12 Gew.-% eines Gemisches aus mittelkettigen Fettsäuretriglyceriden (MCT) und langkettigen Fettsäuretriglyceriden (LCT) als Lipid, 71,5 bis 93 Gew.-% Wasser und 0,3 bis 3,0 Gew.-% Emulgator mit einem HLB-Wert von mindestens 13 und ausgewählt aus der Gruppe Saccharosefettsäureester, Glycerinfettsäureester, Polyoxyethylensorbitanfettsäureester und einem Emulgatorgemisch aus dem Emulgator und Sojabohnenlecithin enthält, und daß der Emulgator einen pH-Wert von 6,5 bis 8,5 besitzt:

| Aminosäure | Menge (Gew.-%) |
| --- | --- |
| L-Isoleucin | 4,5 - 13,4 |
| L-Leucin | 6,7 - 20,1 |
| L-Lysin | 3,8 - 15,2 |
| L-Methionin | 3,0 - 16,4 |
| L-Phenylalanin | 3,1 - 16,4 |
| L-Threonin | 3,2 - 9,6 |
| L-Tryptophan | 0,9 - 3,7 |
| L-Valin | 5,2 - 15,6 |
| L-Alanin | 0 - 9,6 |
| L-Arginin | 0 - 12,6 |
| L-Asparaginsäure | 0 - 12,2 |
| L-Glutaminsäure | 0 - 12,9 |
| L-Glutamin | 0 - 16,2 |
| Aminoessigsäure (Glycin) | 0 - 4,1 |
| L-Histidin | 0 - 6,2 |
| L-Prolin | 0 - 5,3 |
| L-Serin | 0 - 9,7 |
| L-Tyrosin | 0 - 0,9 |

2. Nährmittelemulsion nach Anspruch 1, dadurch **gekennzeichnet,** daß das Aminosäurepräparat die folgende Aminosäurezusammensetzung besitzt, worin die Menge jeder Aminosäure als Gewichtsprozent ausgedrückt ist und als freie Aminosäuren, bezogen auf die Gesamtaminosäuren, berechnet ist.

44

| Aminosäure | Menge (Gew.-%) |
|---|---|
| L-Isoleucin | 4,5 - 13,4 |
| L-Leucin | 6,7 - 20,1 |
| L-Lysin | 3,8 - 11,6 |
| L-Methionin | 3,3 - 7,7 |
| L-Phenylalanin | 3,1 - 9,5 |
| L-Threonin | 3,2 - 9,6 |
| L-Tryptophan | 0,9 - 2,7 |
| L-Valin | 5,2 - 15,6 |
| L-Alanin | 3,2 - 9,6 |
| L-Arginin | 0,9 - 2,7 |
| L-Asparaginsäure | 1,6 - 5,0 |
| L-Glutaminsäure | 4,3 - 12,9 |
| Aminoessigsäure (Glycin) | 1,3 - 4,1 |
| L-Histidin | 1,0 - 3,2 |
| L-Prolin | 1,5 - 4,5 |
| L-Serin | 2,7 - 8,1 |

3. Nährmittelemulsion nach Anspruch 1, dadurch **gekennzeichnet,** daß das Aminosäurepräparat die folgende Aminosäurezusammensetzung besitzt, worin die Menge jeder Aminosäure als Gewichtsprozent ausgedrückt ist und als freie Aminosäuren, bezogen auf die Gesamtaminosäuren, berechnet ist.

| Aminosäure | Menge (Gew.-%) |
|---|---|
| L-Isoleucin | 8,0 - 13,4 |
| L-Leucin | 12,5 - 20,1 |
| L-Lysin | 6,9 - 8,5 |
| L-Methionin | 4,6 - 5,6 |
| L-Phenylalanin | 5,6 - 6,9 |
| L-Threonin | 3,2 - 7,0 |
| L-Tryptophan | 1,6 - 2,0 |
| L-Valin | 5,2 - 11,4 |
| L-Alanin | 5,8 - 7,0 |
| L-Arginin | 7,6 - 9,2 |
| L-Asparaginsäure | 3,0 - 3,6 |
| L-Glutaminsäure | 7,7 - 9,5 |
| Aminoessigsäure (Glycin) | 2,4 - 3,0 |
| L-Histidin | 1,9 - 2,3 |
| L-Prolin | 2,7 - 3,3 |
| L-Serin | 4,9 - 5,9 |

4. Nährmittelemulsion nach einem der Ansprüche 1 bis 3, dadurch **gekennzeichnet,** daß ein Teil der Aminosäuren, aus denen das Aminosäurepräparat besteht, in der Form von Di- bis Pentapeptiden verwendet wird.

5. Nährmittelemulsion nach einem der Ansprüche 1 bis 4, dadurch **gekennzeichnet,** daß der Emulagtor ein Gemisch aus Saccharosefettsäureester und/oder Glycerinfettsäureester mit einem HLB-Wert von nicht weniger als 13 und Sojabohnenlecithin ist, wobei das Verhältnis (Gew.-%) von Saccharosefettsäureester und/oder Glycerinfettsäureester zu Sojabohnenlecithin 75 - 90 : 10 - 25 beträgt.

6. Nährmittelemulsion nach einem der Ansprüche 1 bis 5, dadurch **gekennzeichnet,** daß das Lipid ein Gemisch aus mittelkettigen Fettsäuretriglyceriden (MCT) und langkettigen Fettsäuretriglyceriden (LCT) ist, wobei das Verhältnis (Gew.-%) des zuerst genannten zu dem zuletzt genannten 50 - 90 : 10 - 50 beträgt.

7. Verfahren zur Herstellung einer Nährmittelemulsion nach einem der Ansprüche 1 bis 6, dadurch **gekennzeichnet,** daß man in Stufen ein Lipid oder, sofern erforderlich, ein Gemisch aus einem Lipid und Sojabohnenlecithin zu einem Gemisch aus Wasser und einem Emulgator hinzufügt, wobei der Emulgator einen HLB-Wert von mindestens 13 besitzt und aus der Gruppe Saccharosefettsäureester, Glycerinfettsäureester, Polyoxyethylenglycerinfettsäureester und Polyoxyethylensorbitanfettsäureester ausgewählt ist; das Gemisch voremulgiert; eine wässrige Lösung eines Aminosäurepräparats zu der groben emulgierten Flüssigkeit hinzufügt; und das Gemisch vollständig emulgiert.

**Revendications**

1. Emulsion nutritive contenant en tant que composant essentiel, une préparation d'acides aminés, un lipide, de l'eau et un émulsifiant, qui est caractérisée en ce que l'émulsion comprend 4 à 15% en poids de la préparation d'acides aminés ayant la composition d'acides aminés suivante dans laquelle la quantité de chaque acide aminé est exprimée en pourcentage en poids, 3 à 12% en poids d'un mélange de triglycéride d'acide gras à chaîne moyenne (MCT) et de triglycéride d'acide gras à chaîne longue (LCT) constituant le lipide, 71,5 à 93% en poids d'eau et 0,3 à 3,0% en poids de l'émulsifiant ayant un HLB d'au moins 13 et choisi dans le groupe consistant en ester d'acide gras de sucrose, ester d'acide gras de glycérine, ester d'acide gras de polyoxyéthylène sorbitane et un mélange d'émulsifiants dudit émulsifiant et de lécithine de soja et en ce que l'émulsifiant a un pH de 6,5, à 8,5:

| Acide aminé | Quantité (% en poids) |
|---|---|
| L-isoleucine | 4,5-13,4 |
| L-leucine | 6,7-20,1 |
| L-lysine | 3,8-15,2 |
| L-méthionine | 3,0-16,4 |
| L-phénylalanine | 3,1-16,4 |
| L-thréonine | 3,2- 9,6 |
| L-tryptophane | 0,9- 3,7 |
| L-valine | 5,2-15,6 |
| L-alanine | 0 - 9,6 |
| L-arginine | 0 -12,6 |
| Acide L-aspartique | 0 -12,2 |
| Acide L-glutamique | 0 - 12,9 |
| L-glutamine | 0 - 16,2 |
| Acide aminoacétique (glycine) | 0 - 4,1 |
| L-histidine | 0 - 6,2 |
| L-proline | 0 - 5,3 |
| L-sérine | 0 - 9,7 |
| L-tyrosine | 0 - 0,9 |

2. Emulsion nutritive suivant la revendication 1, dans laquelle la préparation d'acides aminés a la composition d'acides aminés suivante dans laquelle la quantité de chaque acide aminé est exprimée en pourcentage en poids et calculée en tant qu'acide aminé libre par rapport au total des acides aminés :

EP 0 312 612 B1

| Acide aminé | Quantité (% en poids) |
|---|---|
| L-isoleucine | 4,5-13,4 |
| L-leucine | 6,7-20,1 |
| L-lysine | 3,8-11,6 |
| L-méthionine | 3,3- 7,7 |
| L-phénylalanine | 3,1- 9,5 |
| L-thréonine | 3,2- 9,6 |
| L-tryptophane | 0,9- 2,7 |
| L-valine | 5,2-15,6 |
| L-alanine | 3,2- 9,6 |
| L-arginine | 0,9- 2,7 |
| Acide L-aspartique | 1,6- 5,0 |
| Acide L-glutamique | 4,3-12,9 |
| Acide aminoacétique (glycine) | 1,3- 4,1 |
| L-histidine | 1,0- 3,2 |
| L-proline | 1,5- 4,5 |
| L-sérine | 2,7- 8,1 |

3. Emuision nutritive suivant la revendication 1, dans laquelle la préparation d'acides aminés a la composition d'acides aminés suivante dans laquelle la quantité de chaque acide aminé est exprimée en pourcentage en poids et calculée en tant qu'acide aminé libre par rapport au total des acides aminés :

| Acide aminé | Quantité (% en poids) |
|---|---|
| L-isoleucine | 8,0-13,4 |
| L-leucine | 12,5-20,1 |
| L-lysine | 6,9- 8,5 |
| L-méthionine | 4,6- 5,6 |
| L-phénylalanine | 5,6- 6,9 |
| L-thréonine | 3,2- 7,0 |
| L-tryptophane | 1,6- 2,0 |
| L-valine | 5,2-11,4 |
| L-alanine | 5,8- 7,0 |
| L-arginine | 7,6- 9,2 |
| Acide L-aspartique | 3,0- 3,6 |
| Acide L-glutamique | 7,7- 9,5 |
| Acide aminoacétique (glycine) | 2,4- 3,0 |
| L-histidine | 1,9- 3,2 |
| L-proline | 2,7- 3,3 |
| L-sérine | 4,9- 5,9 |

4. Emulsion nutritive suivant l'une quelconque des revendications 1 à 3, dans laquelle une partie des acides aminés constituant cette préparation d'acides aminés est utilisée sous la forme de di- à penta-peptides.

5. Emulsion nutritive suivant l'une quelconque des revendications 1 à 4, dans laquelle l'émulsifiant est un mélange d'ester d'acide gras de sucrose et/ou d'ester d'acide gras de glycérine ayant un HLB d'au moins 13 et de lécithine de soja, le rapport en pourcentage pondéral de l'ester d'acide gras de sucrose et/ou de l'ester d'acide gras de glycérine à la lécithine de soja étant de 75 à 90 : 10 à 25.

6. Emulsion nutritive suivant l'une quelconque des revendications 1 à 5, dans laquelle le lipide est un mélange de triglycéride d'acide gras à chaîne moyenne (MCT) et de triglycéride d'acide gras à chaîne longue (LCT), le rapport en pourcentage pondéral du premier au dernier étant de 50 à 90 : 10 à 50.

47

7. Procédé pour préparer une émulsion nutritive suivant l'une quelconque des revendications 1 à 6, caractérisé en ce qu'il comprend les étapes d'addition d'un lipide ou, lorsque cela est requis, d'un mélange de lipide et de lécithine de soja à un mélange d'eau et d'un émulsifiant, l'émulsifiant ayant un HLB d'au moins 13 et étant choisi dans le groupe consistant en ester d'acide gras de sucrose, ester d'acide gras de glycérine, ester d'acide gras de polyoxyéthylène glycérine et ester d'acide gras de polyoxyéthylène sorbitane; de soumission du mélange à une pré-émulsification; d'addition d'une solution aqueuse d'une préparation d'acides aminés au liquide grossièrement émulsifié; et d'émulsification complète du mélange.